(19) **Europäisches Patentamt European Patent Office Office européen des brevets**

(11) **EP 4 534 205 A1**

(12) **EUROPEAN PATENT APPLICATION**

(43) Date of publication:
**09.04.2025 Bulletin 2025/15**

(21) Application number: **23201239.3**

(22) Date of filing: **02.10.2023**

(51) International Patent Classification (IPC):
**B01L 7/00** (2006.01)   **G01N 35/00** (2006.01)

(52) Cooperative Patent Classification (CPC):
**B01L 7/52;** B01L 2200/143; B01L 2200/147;
B01L 2200/148; B01L 2300/027; B01L 2300/1822;
B01L 2300/1844; B01L 2300/1894

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicants:
• **Roche Diagnostics GmbH**
  **68305 Mannheim (DE)**
  Designated Contracting States:
  **DE**
• **F. Hoffmann-La Roche AG**
  **4070 Basel (CH)**
  Designated Contracting States:
  **AL AT BE BG CH CY CZ DK EE ES FI FR GB GR HR HU IE IS IT LI LT LU LV MC ME MK MT NL NO PL PT RO RS SE SI SK SM TR**

(72) Inventors:
• **Dobrzynski, Michal Karol**
  **6343 Rotkreuz ZG (CH)**
• **Federer, Paul Ivo**
  **6343 Rotkreuz ZG (CH)**
• **Leitao, Ivo Daniel Vicente**
  **6343 Rotkreuz ZG (CH)**
• **Vollenweider, Stefan**
  **6343 Rotkreuz ZG (CH)**

(74) Representative: **Altmann Stößel Dick Patentanwälte PartG mbB Theodor-Heuss-Anlage 2 68165 Mannheim (DE)**

(54) **COMPUTER IMPLEMENTED METHOD FOR PERFORMANCE VERIFICATION OF A THERMAL BLOCK CYCLER UNIT**

(57)    A computer-implemented method for performance verification of a thermal block cycler unit (110) for automated thermal treatment of at least one sample is proposed. The thermal block cycler unit (110) comprises at least one thermal block (118) configured for receiving at least one sample vessel. The thermal block cycler unit (110) further comprises a plurality of hardware modules (120). The hardware modules (120) comprise a plurality of heating and/or cooling elements and at least one temperature sensor (122) The method comprises consecutively functional testing of the hardware modules, wherein an order of tests is defined considering the following criteria

- every consecutive test uses for testing a thermal energy of its preceding test and/or a status of the thermal block cycler unit (110) of its preceding test, and

- energy consumption is balanced between the steps in such a way that a difference between an input temperature before consecutively testing the hardware modules (120) and an output temperature after consecutively testing the hardware modules (120) is minimized.

Fig. 2

**Description**

Technical Field

The invention relates to a computer-implemented method for performance verification of a thermal block cycler unit, a laboratory system, a computer program, a computer-readable storage medium and a non-transient computer-readable medium. The method and devices can be used in the field of biochemical analytics or research, or clinical diagnostics or research. The laboratory system, for example, can be used for an automated thermal treatment of samples. Other applications, however, are also feasible.

Background art

A functional test of a hardware module is a common practice in medical devices, ensuring correct operation of an instrument, and collecting date for, e.g. predictive maintenance. Such a functional test of are known e.g. from WO 2022/101870 A1, US 2014/0122006 A1, US 5,602,756 A, EP 2 108 942 A1 US 10,850,283 B2, US 2013/0157376A1, KR 2375707 B1, EP 3 253 492 A2, US 6,703,236 B2, AU 741806 B2, or US 2008/0212643A1.

However, execution of a functional test can have several drawbacks. For example, it reduces effective time during which module or instrument can be used by end-user, it consumes energy, it reduces lifetime of the module or instrument, and, in many cases, it requires end-user to bring instrument to a correct state to start a run.

Problem to be solved

It is therefore desirable to provide methods and devices, which address the abovementioned technical challenges. Specifically, methods, computer programs and devices shall be proposed, which allow for minimizing energy consumption and time during which a thermal block cycler unit is out of use for a user.

Summary

This problem is addressed by a computer-implemented method for performance verification of a thermal block cycler unit, a laboratory system, a computer program, a computer-readable storage medium and a non-transient computer-readable medium with the features of the independent claims. Advantageous embodiments which might be realized in an isolated fashion or in any arbitrary combinations are listed in the dependent claims as well as throughout the specification.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, it shall be noted that the terms "at least one", "one or more" or similar expressions indicating that a feature or element may be present once or more than once typically will be used only once when introducing the respective feature or element. In the following, in most cases, when referring to the respective feature or element, the expressions "at least one" or "one or more" will not be repeated, non-withstanding the fact that the respective feature or element may be present once or more than once.

Further, as used in the following, the terms "preferably", "more preferably", "particularly", "more particularly", "specifically", "more specifically" or similar terms are used in conjunction with optional features, without restricting alternative possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding alternative embodiments of the invention, without any restrictions regarding the scope of the invention and without any restriction regarding the possibility of combining the features introduced in such way with other optional or non-optional features of the invention.

In a first aspect, a computer-implemented method for performance verification of a thermal block cycler unit for automated thermal treatment of at least one sample is disclosed. The thermal block cycler unit comprises at least one thermal block configured for receiving at least one sample vessel. The thermal block cycler unit further comprises a plurality of hardware modules, wherein the hardware modules comprise a plurality of heating and/or cooling elements and at least one temperature sensor.

The term "computer-implemented" as used herein is a broad term and is to be given its ordinary and customary meaning to a person of ordinary skill in the art and is not to be limited to a special or customized meaning. The term specifically may refer, without limitation, to a method involving at least one computer and/or at least one computer network or a cloud. The computer and/or computer network and/or a cloud may comprise at least one processor which is configured for performing at least one of the method steps of the method according to the present invention. Preferably each of the method steps is performed by the computer and/or computer network and/or a cloud. The method may be performed completely automatically, for example without user interaction. The term "automatically" as used herein is a broad term and is to be given its ordinary and customary meaning to a person of ordinary skill in the art and is not to be limited to a special or customized meaning. The term specifically may refer, without limitation, to a process, which is performed completely by means of at least one computer and/or computer network and/or a cloud and/or machine, for example without manual action and/or interaction with a user.

The method may be used in the field of in-vitro diagnostics. The term "in-vitro diagnostics" as used herein is a broad term and is to be given its ordinary and customary meaning to a person of ordinary skill in the art and is not to be limited to a special or customized meaning. For example, in-vitro diagnostics may comprise performing at least one test on a sample, e.g. on a biological sample that has been taken off the human body or animal body. The method may be performed by using a laboratory system, such as in a laboratory or other environment for performing scientific or technological research, experiments and/or measurements. For example, the laboratory system may be part of a clinical laboratory, a medical laboratory, a forensic laboratory or a blood bank.

The term "sample" as used herein is a broad term and is to be given its ordinary and customary meaning to a person of ordinary skill in the art and is not to be limited to a special or customized meaning. The term specifically may refer, without limitation, to a material suspected of containing at least one analyte of interest. The sample may be a biological sample. The term "biological sample" as used herein is a broad term and is to be given its ordinary and customary meaning to a person of ordinary skill in the art and is not to be limited to a special or customized meaning. The term specifically may refer, without limitation, to at least one biological material that may potentially comprise at least one analyte of interest. The sample can be derived from any biological source, such as a physiological fluid, including, blood, saliva, ocular lens fluid, cerebral spinal fluid, sweat, urine, milk, ascites fluid, mucous, synovial fluid, peritoneal fluid, amniotic fluid, tissue, cells or the like. For example, the biological sample may be or may comprise an aliquot of a substance such as a biological compound. For example, the biological sample may be or may comprise at least one biological specimen, such as one or more of: blood; blood serum; blood plasma; urine; saliva. The biological sample may be a liquid sample. For example, the liquid sample may be or may comprise at least one pure liquid, such as a liquid substance and/or a solution containing one or more liquid substances, comprising the biological substance. The liquid sample may be or may comprise a liquid mixture, such as a suspension, an emulsion and/or a dispersion of biological substances. The biological sample may be a solid sample, for example, at least one sample of tissue. The sample can be pretreated prior to use, such as preparing plasma from blood, diluting viscous fluids, lysis or the like; methods of treatment can involve filtration, distillation, concentration, inactivation of interfering components, and the addition of reagents. A sample may be used directly as obtained from the source or following a pretreatment to modify the character of the sample, e.g. after being diluted with another solution or after having being mixed with reagents e.g. to carry out one or more diagnostic assays like e.g. clinical chemistry assays, immunoassays, coagulation assays, nucleic acid testing, etc.. The term "sample" as used herein is therefore not only used for the original sample but also relates to a sample which has already been processed such as pipetted, diluted, mixed with reagents, enriched, having been purified, having been amplified and the like. As used herein, the term "analyte" may refer to the compound or composition to be detected or measured.

The term "thermal block cycler unit", also denoted as thermocycler, PCR machine or DNA amplifier, as used herein is a broad term and is to be given its ordinary and customary meaning to a person of ordinary skill in the art and is not to be limited to a special or customized meaning. The term specifically may refer, without limitation, to a device configured for performing at least one cyclic temperature change, e.g. a plurality of predefined temperature cycles. The thermal block cycler may be configured for performing temperature cycles, e.g. for temperature-sensitive reactions, e.g. amplification reactions such as for polymerase chain reaction (PCR). For example, the thermal block cycler unit may be designed as described in EP 1 710 017 A1, which is incorporated herein in entirety by reference. Thus, for example, the thermal block cycler unit may comprise a thermal block, at least one thermo-electric cooler, at least one vapor chamber mount, and a heat sink.

The thermal block cycler may comprise at least one thermal block. The thermal block may be a solid state device disposed to have a good thermal conductance. There is a plurality of materials known to someone skilled in the art that have a good thermal conductance and without being bound to theory, most materials having a good electrical conductance are good thermal conductors, too. Therefore, materials like copper, aluminum, silver or graphite are suitable for the thermal block. On the other hand, also plastics and ceramics may have sufficient thermal conductance to be used as material for the thermal block.

The thermal block cycler unit may further comprise a heat sink configured for dissipating heat. In general, a heat sink is made out of a thermally conductive material analogous to the thermal block outlined before. Therefore, heat sinks are mostly made out of metal, preferably out of aluminum or silver. Alternatively, heat sinks may be formed out of plastics and ceramics, if only a good thermal conductance is realized. In order to realize a maximum dissipation of heat, heat sinks are disposed to provide a large surface to-volume ratio. This can be realized by an assembly of fins arranged on a base plate. A large surface-to-volume ratio reduces the heat transfer resistance between the heat sink and the surrounding air.

The term "thermal treatment" as used herein is a broad term and is to be given its ordinary and customary meaning to a person of ordinary skill in the art and is not to be limited to a special or customized meaning. The term specifically may refer, without limitation, to at least one processing step on the sample comprising at least one temperature change such as heating and/or cooling. The temperature change may comprise alternately heating and cooling the sample. The thermal treatment may comprise raising and/or lowering the temperature. The temperature change may be performed continuously or non-continuously in discrete pre-defined steps.

The thermal block is configured for receiving at least one sample vessel. The thermal block cycler, in particular the thermal block, may comprise at least one sample block configured for receiving at least one sample vessel. The sample block may be designed integral with the thermal block.

The term "sample vessel" as used herein is a broad term and is to be given its ordinary and customary meaning to a person of ordinary skill in the art and is not to be limited to a special or customized meaning. The term specifically may refer, without limitation, to a device configured for storing and/or accommodating at least one sample. The sample vessel may be or may comprise one or more of at least one tube shaped container, at least one rack, at least one puck or at least one slide. For example, the sample vessel may comprise at least one multiwell plate. The term "multiwell plate" as used herein is a broad term and is to be given its ordinary and customary meaning to a person of ordinary skill in the art and is not to be limited to a special or customized meaning. The term specifically may refer, without limitation, to any plate-shaped device configured to permanently or temporarily store a sample, e.g. a small amount of a fluid. A small amount in this respect is to be understood as a quantity of fluid in the range of nl to ml such as 10 nl to 100 ml, preferably 0.1 $\mu$l to 10 ml and even more preferably 0.1 $\mu$l to 5 ml. Basically, the design of the multiwell plate may depend on the respective application. The multiwell plate may be designed as a device for storing a single fluid sample or a plurality of fluid samples. Similarly, the geometry of a respective storage area of the multiwell plate may depend on the respective application of the multiwell plate. The storage area may be designed as a well, channel, depression, recess or the like. For example, the multiwell plate may comprise a plurality of wells. The term "multiwell plate" as used herein may refers to a flat plate with multiple "wells" used as small test tubes. Such a multiwell plate is also known as microtiter plate. The microtiter plate has become a standard tool in analytical research and clinical diagnostic testing laboratories. A multiwell plate typically may have 6, 24, 96, 384 or 1536 sample wells, e.g. arranged in a 2:3 rectangular matrix. Wells can be either circular or square. The multiwell plate may be configured as a processing plate. The processing plate may be configured for undergoing at least one sample processing step. The sample processing may comprise one or more of sample specific steps, aliquoting, heating, cooling, magnetic separation, mixing, washing, elution, diluting, chemical reacting, lysing samples, capturing nucleic acids, releasing nucleic acids, extracting nucleic acids or the like. The sample vessel may be a plastic vessel. For example, the sample vessel may be designed for permitting an optimal heat transfer.

The term "sample block" as used herein is a broad term and is to be given its ordinary and customary meaning to a person of ordinary skill in the art and is not to be limited to a special or customized meaning. The term specifically may refer, without limitation, to a block shaped constructional member comprising at least one chamber and/or recess configured for receiving the sample vessel. The sample block may be configured for achieving fast temperature changes and uniform temperature throughout the block. After the insertion of the sample vessel into the sample block, the temperature of the sample block may be changed, e.g. raised and/or lowered according to the predefined temperature cycle(s).

The term "performance" as used herein is a broad term and is to be given its ordinary and customary meaning to a person of ordinary skill in the art and is not to be limited to a special or customized meaning. The term specifically may refer, without limitation, to a measure characterizing thermal behavior of the thermal block cycler unit. The performance may be characterized as good or poor thermal performance. A good thermal performance may relate to a performance within the manufacturer's specifications. A poor thermal performance may relate to a performance deviating from the manufacturer's specifications. The performance may be characterized with respect to at least one parameter such as at least one parameter selected from the group consisting of a heating rate, a cooling rate, a temperature distribution, thermal uniformity, thermal accuracy, thermal resolution, energy efficiency (thermal losses), and the like. The performance of the thermal block cycler unit may be influenced by performance of individual elements of the thermal block cycler unit such as the hardware modules of the thermal block cycler unit.

The term "performance verification" as used herein is a broad term and is to be given its ordinary and customary meaning to a person of ordinary skill in the art and is not to be limited to a special or customized meaning. The term specifically may refer, without limitation, to quality control of performance of the thermal block cycler unit, such as at least one process for verifying good thermal performance of the thermal block cycler unit.

The method steps may be performed in the given order or may be performed in a different order. Further, one or more additional method steps may be present which are not listed. Further, one, more than one or even all of the method steps may be performed repeatedly.

The method comprises consecutively functional testing of the hardware modules, wherein an order of tests is defined considering the following criteria

- every consecutive test uses for testing a thermal energy of its preceding test and/or a status of the thermal block cycler unit of its preceding test, and
- energy consumption is balanced between the steps in such a way that a difference between an input temperature before consecutively testing the hardware modules and an output temperature after consecutively testing the hardware modules is minimized.

The term "functional testing" as used herein is a broad term and is to be given its ordinary and customary meaning to a person of ordinary skill in the art and is not to be limited to a special or customized meaning. The term specifically may refer, without limitation, to testing of at least one basic function of the respective hardware module such as if the hardware module is working at all, and/or testing the respective hardware module against its respective specification. The term "consecutively" functional testing as used herein is a broad term and is to be given its ordinary and customary meaning to a person of ordinary skill in the art and is not to be limited to a special or customized meaning. The term specifically may refer, without limitation, to sequential functional testing the hardware modules, e.g. all hardware modules of the thermal block cycler unit. The functional testing may comprise individually testing the respective hardware module or groups of hardware modules, e.g. of the same kind.

The use of the above-listed criteria allow for energy efficiency and speed. These two objectives can be achieved by setting order of the test steps such that every consecutive step benefits from the thermal energy and/or a thermocycler state, of its preceding step. The amount of the energy put into the system can therefore be minimized, contributing to the reduction of execution time. Moreover, the energy can be balanced between the steps in such a way that the difference between the starting temperature of the test (temperature before the test start) and the finish temperature of the test (temperature after the test finishes) is minimized. This can allow reducing the time during which the thermocycler is out of use for an end-user.

The term "use for testing a thermal energy of its preceding test" as used herein is a broad term and is to be given its ordinary and customary meaning to a person of ordinary skill in the art and is not to be limited to a special or customized meaning. The term specifically may refer, without limitation, to using the thermal energy as starting point for the test and/or as test condition during the test. The term "status of the thermal block cycler unit" as used herein is a broad term and is to be given its ordinary and customary meaning to a person of ordinary skill in the art and is not to be limited to a special or customized meaning. The term specifically may refer, without limitation, to any state of at least one hardware module of the thermal block cycler unit determined and/or set and/or reached during and/or by a previous test. The status may be a status of the hardware module tested during the preceding test and/or the status of the hardware modules tested so far during performing of the method. For example, the status may be a thermal state of the thermal block cycler unit, e.g. at the end of the preceding test. The thermal state may be used as starting point for the test and/or as test condition during the test. For example, the status may be a test result of the preceding test, e.g. a pass or fail state, e.g. with respect to at least one specified acceptance criteria. For example, a next test step may not be executed if one or more previous steps failed. For example, the thermal block will not be heated if the temperature sensors are detected to be nonfunctional and/or if a cooling system has a broken pump. The term "use for testing a status of the thermal block cycler unit of its preceding test" as used herein is a broad term and is to be given its ordinary and customary meaning to a person of ordinary skill in the art and is not to be limited to a special or customized meaning. The term specifically may refer, without limitation, to considering the status of the thermal block cycler unit, e.g. a test result, and/or as a setup for the next test. For example, the status may be used as starting point for the test and/or as test condition during the test.

The term "energy consumption" as used herein is a broad term and is to be given its ordinary and customary meaning to a person of ordinary skill in the art and is not to be limited to a special or customized meaning. The term specifically may refer, without limitation, to any energy applied to the thermal block cycler unit for performing the method for performance verification such as electric energy for heating and/or cooling.

The term "to balance" as used herein is a broad term and is to be given its ordinary and customary meaning to a person of ordinary skill in the art and is not to be limited to a special or customized meaning. The term specifically may refer, without limitation, to ensuring having a minimum overall temperature gradient, e.g. between the input temperature and the output temperature before and after consecutively testing the hardware modules. The balancing may comprise ensuring having a minimum temperature gradient between an output temperature of a test of a hardware module and an input temperature of the consecutive test or a hardware module. A temperature profile over the method steps for performance verification may be as isothermal as possible. The balancing may comprise determining, e.g. computing, a plurality of potential test sequences for functional tests of the hardware modules and selecting the test sequence having an order of the functional tests of the hardware modules fulfilling this requirement.

The performance verification may be part of an autodiagnostic workflow of the thermal block cycler unit. For example, the performance verification may be part of a workflow for predictive maintenance. For example, the performance verification may be part of a workflow for verifying a calibration of the thermal block cycler unit. The performance verification may be performed at start of operation, e.g. during an initial setup and/or each time the thermal block cycler unit is turned on. The method may be performed in regular time intervals and/or upon demand by the user. The performance verification may be performed automatically and repeatedly at predefined times, e.g. every 10 runs, or once a day, or during every instrument startup. The performance verification may be automatically triggered and performed in case of a deviation in performance is observed for at least one hardware module, such as absence of signals from a temperature sensor, malfunction in electronics and the like. For example, the performance test may be triggered by detection of at least one deviation in heating and/or cooling ramps from any expected profiles, to detect the root cause. For example, the performance test may be triggered in case of high or low heatsink temperature during a run. The performance verification may be automatically triggered and/or executed by at least one control unit. Additionally, the performance verification may be performed upon user-demand, e.g. via at least one user interface.

The term "control unit" as used herein is a broad term and is to be given its ordinary and customary meaning to a person of ordinary skill in the art and is not to be limited to a special or customized meaning. The term specifically may refer, without limitation, to an arbitrary device or system configured for performing the named operations, preferably by using at least one data processing device and, more preferably, by using at least one processor and/or at least one application-specific integrated circuit. Thus, as an example, the at least one control unit may comprise at least one data processing device having a software code stored thereon comprising a number of computer commands. The control unit may provide one or more hardware elements for performing one or more of the named operations and/or may provide one or more processors with software running thereon for performing one or more of the named operations. The control unit may comprise one or more programmable devices such as one or more computers, application-specific integrated circuits (ASICs), Digital Signal Processors (DSPs), or Field Programmable Gate Arrays (FPGAs). Additionally or alternatively, however, the control unit may also fully or partially be embodied by hardware. The thermal block cycler unit may comprise and/or may be connected to at least one control unit.

The term "processor" or "processing device" as used herein is a broad term and is to be given its ordinary and customary meaning to a person of ordinary skill in the art and is not to be limited to a special or customized meaning. The term specifically may refer, without limitation, to an arbitrary logic circuitry configured for performing basic operations of a computer or system, and/or, generally, to a device which is configured for performing calculations or logic operations. In particular, the processor may be configured for processing basic instructions that drive the computer or system. As an example, the processor may comprise at least one arithmetic logic unit (ALU), at least one floating-point unit (FPU), such as a math co-processor or a numeric co-processor, a plurality of registers, specifically registers configured for supplying operands to the ALU and storing results of operations, and a memory, such as an L1 and L2 cache memory. In particular, the processor may be a multi-core processor.

Specifically, the processor may be or may comprise a central processing unit (CPU). Additionally or alternatively, the processor may be or may comprise a microprocessor, thus specifically the processor's elements may be contained in one single integrated circuitry (IC) chip. Additionally or alternatively, the processor may be or may comprise one or more application-specific integrated circuits (ASICs) and/or one or more field-programmable gate arrays (FPGAs) and/or one or more tensor processing unit (TPU) and/or one or more chip, such as a dedicated machine learning optimized chip, or the like. The processor specifically may be configured, such as by software programming, for performing one or more evaluation operations.

The method may comprise displaying a result of the performance verification via at least one communication interface and/or at least one user interface. Additionally or alternatively, the method may comprise reporting the result of the performance verification via the communication interface to a laboratory information management system (LIMS). The method may comprise at least one action depending on the result of the performance verification such as one or more of continuing operation, repeating at least one step of the performance verification, issuing a warning, issuing an information to the manufacturer or a service provider, replacing a hardware module, requesting thermal calibration, calling service, controlling cooling agent (in case of liquid cooling), controlling electrical connection to the heating module.

The term "communication interface" as used herein is a broad term and is to be given its ordinary and customary meaning to a person of ordinary skill in the art and is not to be limited to a special or customized meaning. The term specifically may refer, without limitation, to an item or element forming a boundary configured for transferring information. The communication interface may be configured for transferring information from a computational device, e.g. a computer, such as to send or output information, e.g. onto another device. Additionally or alternatively, the communication interface may be configured for transferring information onto a computational device, e.g. onto a computer, such as to receive information. The communication interface may specifically provide means for transferring or exchanging information. In particular, the communication interface may provide a data transfer connection, e.g. Bluetooth, NFC, inductive coupling or the like. As an example, the communication interface may be or may comprise at least one port comprising one or more of a network or internet port, a USB-port and a disk drive. The communication interface may be at least one web interface. The term "user interface" as used herein is a broad term and is to be given its ordinary and customary meaning to a person of ordinary skill in the art and is not to be limited to a special or customized meaning. The term may refer, without limitation, to a feature of the laboratory instrument which is configured for interacting with its environment, such as for the purpose of unidirectionally or bidirectionally exchanging information, such as for exchange of one or more of data or commands. For example, the user interface may be configured to share information with a user and to receive information by the user. The user interface may be a feature to interact visually with a user, such as a display, or a feature to interact acoustically with the user. The user interface, as an example, may comprise one or more of: a graphical user interface; a data interface, such as a wireless and/or a wire-bound data interface.

The hardware modules comprise a plurality of heating and/or cooling elements and at least one temperature sensor.

The hardware modules may comprise at least one temperature sensor. The term "sensor" as used herein is a broad term and is to be given its ordinary and customary meaning to a person of ordinary skill in the art and is not to be limited to a special or customized meaning. The term specifically may refer, without limitation, to an arbitrary element or device configured for detecting at least one condition or for measuring at least one measurement variable. The term "temperature sensor" as used herein is a broad term and is to be given its ordinary and customary meaning to a person of ordinary skill in the art and is not to be limited to a special or customized meaning. The term specifically may refer, without limitation, to a sensor configured for measuring temperature, e.g. an absolute value and/or a temperature change. The thermal block cycler unit may comprise a plurality of temperature sensors. At least one temperature sensor may be used for absolute temperature control. At least one other temperature sensor may be used for determining temperature homogeneity.

The term "heating and/or cooling element" as used herein is a broad term and is to be given its ordinary and customary meaning to a person of ordinary skill in the art and is not to be limited to a special or customized meaning. The term specifically may refer, without limitation, to a device configured for changing, e.g. raising and/or reducing, its temperature and/or an ambient temperature and/or a temperature of a further device.

For example, the hardware modules comprise one or more of at least one fan, at least one operational pump, at least one at least one thermo-electric cooler, at least one vapor chamber mount.

The hardware modules may comprise at least one fan. The fan may be configured for cooling the thermal block cycler unit during operation. The fan may be configured for providing airflow through the thermal block cycler unit. The airflow may be expelled from the thermal block cycler unit at an opening of a housing of the thermal block cycler unit. The thermal block cycler unit may comprise a plurality of fans.

The hardware modules may comprise at least one operational pump. The thermal block cycler unit may comprise at least one water cooling system with an operational pump and with solid-liquid and liquid-air heat exchangers.

The hardware modules may comprise at least one at least one thermo-electric cooler (TEC). Thermoelectric devices are solid-state heat pumps made from semiconductor materials comprising a series of p-type and n-type semiconductor pairs or junctions sandwiched between ceramic plates. Heat is absorbed by electrons at the cold junction as they pass from a low energy level in a p-type element to a higher energy level in an n-type element. At a hot junction, energy is expelled to e.g. a heat sink as the electrons move from the high-energy n-type element to a low-energy p-type element. A DC power supply may provide the energy to move the electrons through the system. A typical TEC may comprise up to 127 junctions and can pump as much as 120 W of heat. The amount of heat pumped may be proportional to the amount of current flowing through the TEC and therefore, tight temperature control is possible. By reversing the current, TECs can function as heaters or coolers, which can be useful in controlling an object in changing ambient environments or in cycling at different temperatures. Sizes range from 2 to 62 mm, and multiple TECs can be used for greater cooling. Because of the relatively large amount of heat being pumped over a small area, TECs in general require a heat sink to dissipate the heat into the ambient environment. A well known type of TECs is the Peltier elements. The thermal block cycler unit may be driven by a plurality of thermo-electric coolers. For example, the thermal block cycler unit may be driven by six Peltier elements.

The hardware modules may comprise at least one vapor chamber mount. The thermal block cycler unit may comprise a plurality of vapor chamber mounts. For example, the VCM may be designed as described in EP 1 710 017 A1 or WO 2013/075839, which are incorporated herein in entirety by reference. The vapor chamber mount may be configured for providing temperature homogeneity over the thermal block. The VCM may be configured for transporting and distributing heat. The vapor chamber mount may have at least partially a plate like structure. The VCM may comprise a lower shell and an upper shell. At least one gas- and liquid-tight intermediate space may be formed between the lower shell and the upper shell in which a fluid working medium and a porous material is arranged. The porous material may contact the upper shell and/or the lower shell at least in regions, but does not completely fill the intermediate space, forming at least one cavity-like vapor gap. For example, ammonia, water, acetone, or methanol are typically used as working medium. The control unit can modify the thermal conductivity of the VCM e.g. by adjusting a flow rate within the intermediate space.

For example, the method comprises testing the temperature sensor. The testing may comprise an electric test. The electric test may comprise testing if a temperature value is generated by the temperature sensor. The electric test may comprise a basic functional test if the temperature sensor or the different temperature sensors are operating at all, e.g. independent from correctness of the measured temperature. In case a temperature value is generated, e.g. a signal from the temperature sensor is received by the control unit, the temperature sensor has passed the test. Otherwise, the temperature sensor is erroneous. In this case, an error may be issued and/or noted by the control unit.

Additionally or alternatively, for example, the method comprises testing the temperature sensor, wherein the testing comprises testing a response behavior of the temperature sensor. Such a test may also imply testing heating capability of the thermal block. The testing of the response behavior may comprise increasing the temperature to a maximum temperature or decreasing the temperature to a minimum temperature for a predefined time range, reading temperature values generated by the temperature sensor before changing the temperature and after changing the temperature, calculating a difference and comparing the difference to at least one predefined threshold for a minimum temperature change. The threshold for a minimum temperature change may be defined with respect to a thermal design of the heating block assuming a worst case scenario, i.e. the worst ambient thermal conditions. In case the threshold for a minimum temperature change is exceeded or at least reached, the temperature sensor has passed the test. Otherwise, the temperature sensor is erroneous. In this case an error may be issued and/or noted by the control unit.

Additionally or alternatively, the method comprises testing the fan. The testing may comprise testing a fan speed. The testing of the fan speed may comprise setting the fan to maximum, monitoring the fan speed, e.g. by using a fan-internal status signal, and comparing the fan speed to at least one predefined threshold for a maximum fan speed. The threshold may be defined as an expected fan speed according to the fan's datasheet. In case the threshold is exceeded and/or reached, the fan has passed the test. Otherwise, the fan is erroneous. In this case an error may be issued and/or noted by the control unit.

Additionally or alternatively, the method comprises testing the operational pump. The testing may comprise testing a pump speed. The testing of the pump speed may comprise setting the pump to maximum, monitoring the pump speed, e.g. by using its internal status signal, and comparing the pump speed to at least one predefined threshold for a maximum pump speed. The threshold may be defined as an expected pump speed according to the pump's datasheet. In case the threshold is exceeded and/or reached the pump has passed the test. Otherwise the pump is erroneous. In this case an error may be issued and/or noted by the control unit.

Additionally or alternatively, the method comprises testing the thermo-electric cooler (TEC). The testing of the TECs may comprises consecutively testing of a plurality of thermo-electric cooler pairs. The testing may comprise measuring current and/or voltage during heating and cooling. The testing further may comprises comparing the measured current and/or voltage during heating and during cooling to a predefined threshold. The threshold may be defined in accordance with electrical parameters of the TEC assuming a worst-case scenario, e.g. a worst ambient thermal conditions. In case the threshold is exceeded and/or reached the respective thermo-electric cooler pair has passed the test. Otherwise, it is erroneous. In this case an error may be issued and/or noted by the control unit.

Additionally or alternatively, the method comprises testing all thermo-electric coolers. The testing may comprise testing power consumption during heating. The power consumption may be compared to a predefined threshold. The threshold may be defined in accordance with electrical parameters of the TECs assuming a worst-case scenario, e.g. a worst ambient thermal conditions. In case the threshold is exceeded and/or reached the thermo-electric coolers have passed the test. Otherwise at least one of them is erroneous. In this case an error may be issued and/or noted by the control unit.

Additionally or alternatively, the method may comprises testing at least one vapor chamber mount (VCM). The testing may comprise sequentially controlling the thermo-electric coolers and monitoring of a thermal distribution of the vapor chamber mount. The TECs may be spatially arranged between the solid-liquid heat exchanger and the VCM. The TECs may be sequentially controlled and the thermal distribution in the VCM may be monitored. The thermal distribution may be compared to a predefined threshold. The threshold may be defined as maximum deviation from expected temperature uniformity after a defined time. In case the threshold is not exceeded and/or reached the VCMs have passed the test. Otherwise, at least one of them is erroneous. In this case an error may be issued and/or noted by the control unit.

For example, the method comprises the following sequence of tests:

    i. basic electric testing of temperature sensors;
    ii. testing fan;
    iii. testing pump;
    iv. consecutively testing of a plurality of thermo-electric cooler pairs;
    v. testing power consumption of all thermo-electric coolers;
    vi. testing temperature sensor ramp;
    vii. testing vapor chamber mounts,
    viii. at least one closing action comprising one or more of disabling all active elements and reporting data.

This order of tests can allow for fulfilling the above-mentioned criteria. However, other orders may be possible. The method may comprise all steps i. to viii. listed above or one or more tests may be skipped, e.g. the testing of vapor chamber mounts may be skipped in case no vapor chamber mounts are present in the thermal block cycler unit. Moreover, the steps may be performed in the given order or a different order. Further, one or more of the method steps may be performed in parallel and/or in a time overlapping fashion. Further, one or more of the method steps may be performed repeatedly. Further, additional method steps may be present which are not listed.

The tests to be performed and/or the order of tests may depend on the hardware modules present in the thermal block cycler unit. The test to be performed and/or the order of steps may be determined, e.g. automatically.

For example, during or upon initialization of the thermal block cycler unit, e.g. automatically, a test of each hardware module of the thermal block cycler unit may be performed, e.g. in accordance to a test protocol such as predefined by the manufacturer. Said test of each hardware module may comprise determining at least one thermal profile for each test. The thermal profiles for the tests may be analyzed and the tests to be performed and/or the order of tests of the method may be defined considering the mentioned criteria.

The method may be performed completely automatically. For example, each of the steps may be executed by the control unit, by controlling operation of the respective hardware modules. The control unit may be configured for receiving the test results via the communication interface. The control unit may be configured for evaluating of the test results and for issuing errors and/or at least one other action.

The method may comprise testing the complete order of steps or aborting the sequence in case of an error occurs.

The basic electric test of temperature sensors may be performed first. In step i. no energy may be inputted or outputted. This step can be performed fast. In step ii., the temperature may be reduced. In step iii., the temperature reduced. Steps iv. and v. to may be temperature neutral, e.g. the temperature may be increased and reduced. Step iv. may comprise the consecutive substeps of testing a first TEC pair, testing a second TEC pair and testing a third TEC pair. In step v., the power consumption of all TECs may be tested, wherein the temperature is increased. In step vi., the testing of the temperature sensor ramp may be performed and the build up temperature of the previous step can be used to measure temperature sensors heating ramp rates. This step may comprise reversing the TEC to cooling and measure cooling ramp rate. The testing vapor chamber mounts may be performed such that an end temperature is similar to starting temperature of the complete sequence. The closing action may comprise disabling of all active elements and/or reporting data.

In a further aspect, a laboratory system comprising at least one instrument for performing a temperature-dependent reaction comprising a thermal block cycler unit for automated thermal treatment of at least one sample, at least one control unit and at least one communication interface. The laboratory system is configured for performing a method for performance verification according to the present invention such as described above or in more detail below. For details, options and definitions, reference may be made to the method as discussed above.

The term "instrument" as used herein is a broad term and is to be given its ordinary and customary meaning to a person of ordinary skill in the art and is not to be limited to a special or customized meaning. The term specifically may refer, without limitation, to any device configured for performing sample processing steps. For example, the instrument may be a clinical diagnostic analyzer.

The instrument further comprises at least one detection unit. The instrument may comprise in addition to the thermal block cycler unit at least one optical unit, e.g. configured for fluorescence excitation and detection of fluorescence light.

Further disclosed and proposed herein is a computer program including computer-executable instructions for performing the method according to the present invention in one or more of the embodiments enclosed herein when the instructions are executed on a computer or computer network. Specifically, the computer program may be stored on a computer-readable data carrier and/or on a computer-readable storage medium.

As used herein, the terms "computer-readable data carrier" and "computer-readable storage medium" specifically may refer to non-transitory data storage means, such as a hardware storage medium having stored thereon computer-executable instructions. The computer-readable data carrier or storage medium specifically may be or may comprise a storage medium such as a random-access memory (RAM) and/or a read-only memory (ROM).

Thus, specifically, one, more than one or even all of method steps, e.g. of the sequence i. to viii. as indicated above may be performed by using a computer or a computer network, preferably by using a computer program.

Further disclosed and proposed herein is a computer program product having program code means, in order to perform the method according to the present invention in one or more of the embodiments enclosed herein when the program is executed on a computer or computer network. Specifically, the program code means may be stored on a computer-readable data carrier and/or on a computer-readable storage medium.

Further disclosed and proposed herein is a data carrier having a data structure stored thereon, which, after loading into a computer or computer network, such as into a working memory or main memory of the computer or computer network, may execute the method according to one or more of the embodiments disclosed herein.

Further disclosed and proposed herein is a non-transient computer-readable medium including instructions that, when executed by one or more processors, cause the one or more processors to perform the method according to one or more of the embodiments disclosed herein.

Further disclosed and proposed herein is a computer program product with program code means stored on a machine-readable carrier, in order to perform the method according to one or more of the embodiments disclosed herein, when the program is executed on a computer or computer network. As used herein, a computer program product refers to the program as a tradable product. The product may generally exist in an arbitrary format, such as in a paper format, or on a computer-readable data carrier and/or on a computer-readable storage medium. Specifically, the computer program product may be distributed over a data network.

Finally, disclosed and proposed herein is a modulated data signal which contains instructions readable by a computer system or computer network, for performing the method according to one or more of the embodiments disclosed herein.

Referring to the computer-implemented aspects of the invention, one or more of the method steps or even all of the method steps of the method according to one or more of the embodiments disclosed herein may be performed by using a computer or computer network. Thus, generally, any of the method steps including provision and/or manipulation of data may be performed by using a computer or computer network. Generally, these method steps may include any of the method steps, typically except for method steps requiring manual work, such as providing the samples and/or certain aspects of performing the actual measurements.

Specifically, further disclosed herein are:

- a computer or computer network comprising at least one processor, wherein the processor is adapted to perform the method according to one of the embodiments described in this description,
- a computer loadable data structure that is adapted to perform the method according to one of the embodiments described in this description while the data structure is being executed on a computer,
- a computer program, wherein the computer program is adapted to perform the method according to one of the embodiments described in this description while the program is being executed on a computer,
- a computer program comprising program means for performing the method according to one of the embodiments described in this description while the computer program is being executed on a computer or on a computer network,
- a computer program comprising program means according to the preceding embodiment, wherein the program means are stored on a storage medium readable to a computer,
- a storage medium, wherein a data structure is stored on the storage medium and wherein the data structure is adapted to perform the method according to one of the embodiments described in this description after having been loaded into a main and/or working storage of a computer or of a computer network, and
- a computer program product having program code means, wherein the program code means can be stored or are stored on a storage medium, for performing the method according to one of the embodiments described in this description, if the program code means are executed on a computer or on a computer network.

Summarizing and without excluding further possible embodiments, the following embodiments may be envisaged:

Embodiment 1. A computer-implemented method for performance verification of a thermal block cycler unit for automated thermal treatment of at least one sample, wherein the thermal block cycler unit comprises at least one thermal block configured for receiving at least one sample vessel,

wherein the thermal block cycler unit further comprises a plurality of hardware modules, wherein the hardware modules comprise a plurality of heating and/or cooling elements and at least one temperature sensor,
wherein the method comprises consecutively functional testing of the hardware modules, wherein an order of tests is defined considering the following criteria

- every consecutive test uses for testing a thermal energy of its preceding test and/or a status of the thermal block cycler unit of its preceding test, and
- energy consumption is balanced between the steps in such a way that a difference between an input temperature before consecutively testing the hardware modules and an output temperature after consecutively testing the hardware modules is minimized.

Embodiment 2. The method according to the preceding embodiment, wherein the hardware modules comprise one or more of at least one temperature sensor, at least one fan, at least one operational pump, at least one at least one thermo-electric cooler, at least one vapor chamber mount.

Embodiment 3. The method according to the preceding embodiments, wherein the method comprises testing the temperature sensor, wherein the testing comprises an electric test, wherein the electric test comprises testing if a temperature value is generated by the temperature sensor.

**Embodiment 4.** The method according to any one of the two preceding embodiments, wherein the method comprises testing the temperature sensor, wherein the testing comprises testing a response behavior of the temperature sensor, wherein the testing of the response behavior comprises increasing the temperature to a maximum temperature or decreasing the temperature to a minimum temperature for a predefined time range, reading temperature values generated by the temperature sensor before changing the temperature and after changing the temperature, calculating a difference and comparing the difference to at least one predefined threshold for a minimum temperature change, wherein in case the threshold is exceeded and/or reached the temperature sensor has passed the test.

**Embodiment 5.** The method according to any one of the three preceding embodiments, wherein the method comprises testing the fan, wherein the testing comprises testing a fan speed, wherein the testing of the fan speed comprises setting the fan to maximum, monitoring the fan speed, and comparing the fan speed to at least one predefined threshold for a maximum fan speed, wherein in case the threshold is exceeded and/or reached the fan has passed the test.

**Embodiment 6.** The method according to any one of the four preceding embodiments, wherein the method comprises testing the operational pump, wherein the testing comprises testing a pump speed, wherein the testing of the pump speed comprises setting the pump to maximum, monitoring the pump speed, and comparing the pump speed to at least one predefined threshold for a maximum pump speed, wherein in case the threshold is exceeded and/or reached the pump has passed the test.

**Embodiment 7.** The method according to any one of the five preceding embodiments, wherein the method comprises testing the thermo-electric cooler, wherein the testing comprises consecutively testing of a plurality of thermo-electric cooler pairs, wherein the testing comprises measuring current and/or voltage during heating and cooling, wherein the testing further comprises comparing the measured current and/or voltage during heating and during cooling to a predefined threshold, wherein in case the threshold is exceeded and/or reached the respective thermo-electric cooler pair has passed the test.

**Embodiment 8.** The method according to any one of the six preceding embodiments, wherein the method comprises testing all thermo-electric coolers, wherein the testing comprises testing power consumption during heating, wherein the power consumption is compared to a predefined threshold, wherein in case the threshold is exceeded and/or reached the thermo-electric coolers have passed the test.

**Embodiment 9.** The method according to any one of the seven preceding embodiments, wherein the method comprises testing vapor chamber mounts, wherein the testing comprises sequentially controlling the thermo-electric coolers and monitoring of a thermal distribution of the vapor chamber mounts, wherein the thermal distribution is compared to a predefined threshold defined as maximum deviation from expected temperature uniformity after a defined time, wherein in case the threshold is not exceeded and/or reached the vapor chamber mount has passed the test.

**Embodiment 10.** The method according to any one of the eight preceding embodiments, wherein the method comprises the following sequence of tests:

    i. basic electric test of temperature sensors;
    ii. test fan;
    iii. test pump;
    iv. consecutively testing of a plurality of thermo-electric cooler pairs;
    v. testing power consumption of all thermo-electric coolers;
    vi. testing temperature sensor ramp;
    vii. testing vapor chamber mounts,
    viii. at least one closing action comprising one or more of disabling all active elements and reporting data.

**Embodiment 11.** The method according to any one of the preceding embodiments, wherein the method comprises displaying a result of the performance verification via at least one communication interface and/or at least one user interface.

**Embodiment 12.** The method according to the preceding embodiment, wherein the method comprises reporting the result of the performance verification via the communication interface to a LIMS system.

Embodiment 13. The method according to any one of the preceding embodiments, wherein the method is performed in regular time intervals and/or upon demand by the user.

Embodiment 14. The method according to any one of the preceding embodiments, wherein the thermal block cycler unit comprises and/or is connected to at least one control unit.

Embodiment 15. Laboratory system comprising at least one instrument for performing a temperature-dependent reaction comprising a thermal block cycler unit for automated thermal treatment of at least one sample, at least one control unit and at least one communication interface, wherein the laboratory system is configured for performing a method for performance verification according to any one of embodiments 1 to 14.

Embodiment 16. The laboratory system according to the preceding embodiment, wherein the instrument further comprises at least one detection unit.

Embodiment 17. A computer program comprising instructions which, when the program is executed by the laboratory system according to any one of the preceding embodiments referring to a laboratory system, cause the laboratory system to perform the method according to any one of the preceding embodiments referring to a method.

Embodiment 18. A computer-readable storage medium comprising instructions which, when the instructions are executed by the laboratory system according to any one of the preceding embodiments referring to a laboratory system, cause the laboratory system to perform the method according to any one of the preceding embodiments referring to a method.

Embodiment 19. A non-transient computer-readable medium including instructions that, when executed by one or more processors, cause the one or more processors to perform he method according to any one of the preceding embodiments referring to a method.

Short description of the Figures

Further optional features and embodiments will be disclosed in more detail in the subsequent description of embodiments, preferably in conjunction with the dependent claims. Therein, the respective optional features may be realized in an isolated fashion as well as in any arbitrary feasible combination, as the skilled person will realize. The scope of the invention is not restricted by the preferred embodiments. The embodiments are schematically depicted in the Figures. Therein, identical reference numbers in these Figures refer to identical or functionally comparable elements.
In the Figures:

Figure 1    shows an exemplary flowchart of an embodiment of a computer-implemented method for performance verification of a thermal block cycler unit;

Figure 2    shows experimental data, in particular temperature profiles of five runs of performing the method for performance verification according to Figure 1, and

Figure 3    shows an embodiment of a laboratory system.

Detailed description of the embodiments

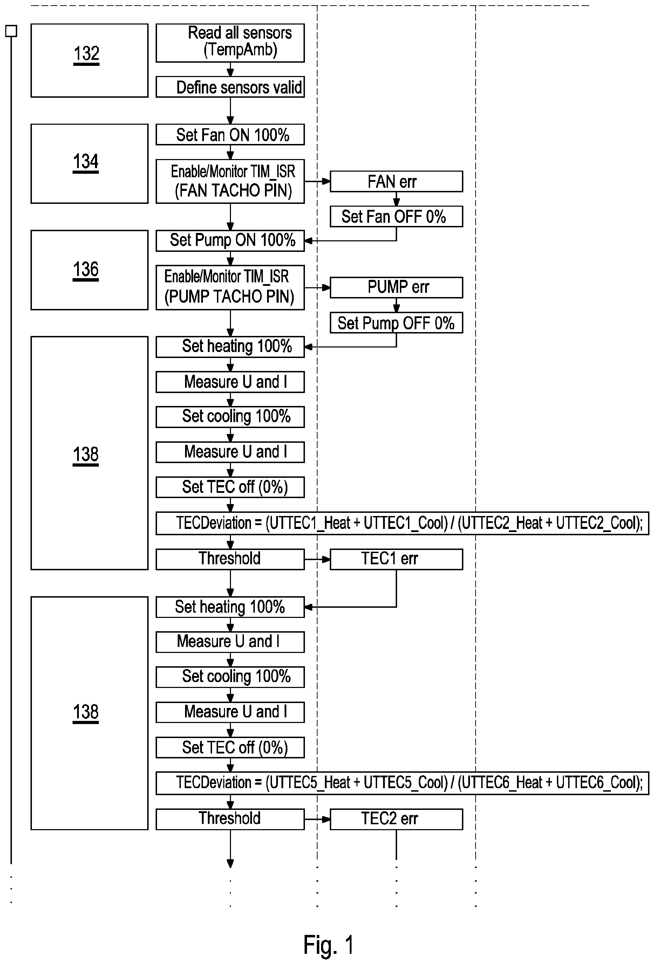

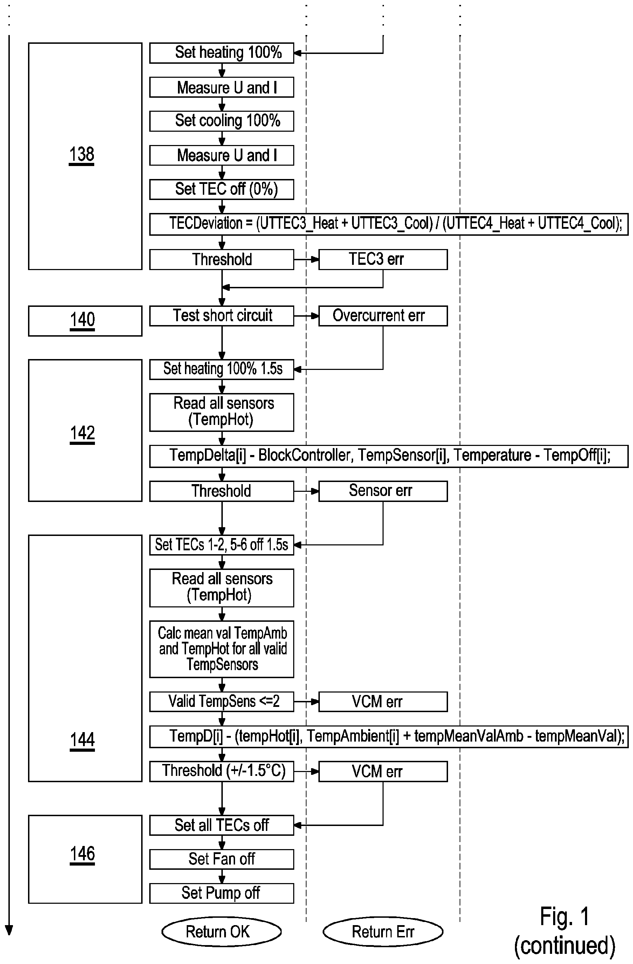

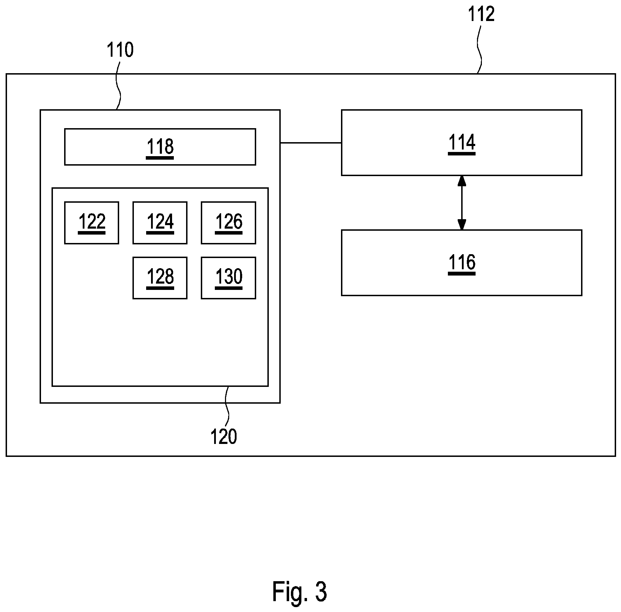

Figure 1 shows an exemplary flowchart of an embodiment of a computer-implemented method for performance verification of a thermal block cycler unit 110 for automated thermal treatment of at least one sample. The thermal block cycler unit 110 may be an element of a laboratory system 112, e.g. as shown in Figure 3. The laboratory system 112 further comprises at least one control unit 114 and at least one communication interface 116.
The method may be used in the field of in-vitro diagnostics. For example, the laboratory system may be part of a clinical laboratory, a medical laboratory, a forensic laboratory or a blood bank. The sample may be a biological sample. The sample can be derived from any biological source, such as a physiological fluid, including, blood, saliva, ocular lens fluid, cerebral spinal fluid, sweat, urine, milk, ascites fluid, mucous, synovial fluid, peritoneal fluid, amniotic fluid, tissue, cells or the like. For example, the biological sample may be or may comprise an aliquot of a substance such as a biological compound. For example, the biological sample may be or may comprise at least one biological specimen, such as one or more of blood; blood serum; blood plasma; urine; saliva. The biological sample may be a liquid sample. For example, the liquid sample may be or may comprise at least one pure liquid, such as a liquid substance and/or a solution containing one or more liquid substances, comprising the biological substance. The liquid sample may be or may comprise a liquid mixture, such as a suspension, an emulsion and/or a dispersion of biological substances. The biological sample may be a solid sample, for example, at least one sample of tissue. The sample can be pretreated prior to use, such as preparing plasma from blood, diluting viscous fluids, lysis or the like; methods of treatment can involve filtration, distillation, concentration, inactivation of interfering components, and the addition of reagents. A sample may be used directly as obtained from the source or following a pretreatment to modify the character of the sample, e.g. after being diluted with another solution or after having being mixed with reagents e.g. to carry out one or more diagnostic assays like e.g. clinical chemistry assays, immunoassays, coagulation assays, nucleic acid testing, etc..

The thermal block cycler unit 110 comprises at least one thermal block 118. The thermal block 118 may be configured for performing temperature cycles, e.g. for temperature-sensitive reactions, e.g. amplification reactions such as for polymerase chain reaction (PCR). The thermal block 118 is configured for receiving at least one sample vessel. The thermal block 118 may comprise at least one sample block configured for receiving at least one sample vessel. The sample vessel may be or may comprise one or more of at least one tube shaped container, at least one rack, at least one puck or at least one slide. For example, the sample vessel may comprise at least one multiwell plate. A multiwell plate typically may have 6, 24, 96, 384 or 1536 sample wells, e.g. arranged in a 2:3 rectangular matrix. Wells can be either circular or square. The sample block may be configured for achieving fast temperature changes and uniform temperature throughout the block. After the insertion of the sample vessel into the sample block, the temperature of the sample block may be changed, e.g. raised and/or lowered according to the predefined temperature cycle(s).

The thermal block cycler unit 110 further comprises a plurality of hardware modules 120. For example, the thermal block cycler unit 110 further comprises at least one temperature sensor 122. The thermal block cycler unit 110 may comprise a plurality of temperature sensors 122. At least one temperature sensor 122 may be used for absolute temperature control. At least one other temperature sensor 122 may be used for determining temperature homogeneity.

Moreover, the hardware modules 120 comprise a plurality of heating and/or cooling elements. For example, the hardware modules comprise one or more of at least one fan 124, at least one operational pump 126, at least one at least one thermo-electric cooler 128, at least one vapor chamber mount 130.

The fan 124 may be configured for cooling the thermal block cycler unit 110 during operation. The fan 124 may be configured for providing airflow through the thermal block cycler unit 110. The airflow may be expelled from the thermal block cycler unit 110 at an opening of a housing of the thermal block cycler unit 110. The thermal block cycler unit 110 may comprise a plurality of fans 124.

The thermal block cycler unit 110 may comprise at least one water cooling system with an operational pump 126 and with solid-liquid and liquid-air heat exchangers.

The thermal block cycler unit 110 may be driven by a plurality of thermo-electric coolers 128, e.g. six Peltier elements.

The vapor chamber mount 130 may be configured for providing temperature homogeneity over the thermal block 118. The thermal block cycler unit 110 may comprise a plurality of vapor chamber mounts 130.

The proposed method allows for performance verification of the thermal block cycler unit 110. The method may be performed completely automatically, for example without user interaction.

The performance may be a measure characterizing thermal behavior of the thermal block cycler unit 110. The performance may be characterized as good or poor thermal performance. A good thermal performance may relate to a performance within the manufacturer's specifications. A poor thermal performance may relate to a performance deviating from the manufacturer's specifications. The performance may be characterized with respect to at least one parameter such as at least one parameter selected from the group consisting of: a heating rate, a cooling rate, a temperature distribution, thermal uniformity, thermal accuracy, thermal resolution, energy efficiency (thermal losses), and the like. The performance of the thermal block cycler unit 110 may be influenced by performance of individual elements of the thermal block cycler unit 110 such as the hardware modules 120 of the thermal block cycler unit 110. The performance verification may comprise a quality control of performance of the thermal block cycler unit 110, such as at least one process for verifying good thermal performance of the thermal block cycler unit 110.

The method steps may be performed in the given order or may be performed in a different order. Further, one or more additional method steps may be present which are not listed. Further, one, more than one or even all of the method steps may be performed repeatedly.

The method comprises consecutively functional testing of the hardware modules 120, wherein an order of tests is defined considering the following criteria

- every consecutive test uses for testing a thermal energy of its preceding test and/or a status of the thermal block cycler unit of its preceding test, and
- energy consumption is balanced between the steps in such a way that a difference between an input temperature before consecutively testing the hardware modules and an output temperature after consecutively testing the hardware modules is minimized.

The functional testing may comprise testing of at least one basic function of the respective hardware module 120 such as if the hardware module is working at all, and/or testing the respective hardware module 120 against its respective specification. The functional testing may comprise individually testing the respective hardware module or groups of hardware modules, e.g. of the same kind.

The use of the above-listed criteria allow for energy efficiency and speed. These two objectives can be achieved by setting order of the test steps such that every consecutive step benefits from the thermal energy and/or a thermocycler state, of its preceding step. The amount of the energy put into the system can therefore be minimized, contributing to the reduction of execution time. Moreover, the energy can be balanced between the steps in such a way that the difference between the starting temperature of a thermocycler (temperature before the test start) and the finish temperature of a thermocycler (temperature after the test finishes) is minimized. This can allow reducing the time during which the thermocycler is out of use for an end-user.

The performance verification may be part of an autodiagnostic workflow of the thermal block cycler unit 110. For example, the performance verification may be part of a workflow for predictive maintenance. For example, the performance verification may be part of a workflow for verifying a calibration of the thermal block cycler unit 110. The performance verification may be performed at start of operation, e.g. during an initial setup and/or each time the thermal block cycler unit 110 is turned on. The method may be performed in regular time intervals and/or upon demand by the user. The performance verification may be performed automatically and repeatedly at predefined times, e.g. every 10 runs, or once a day, or during every instrument startup. The performance verification may be automatically triggered and performed in case of a deviation in performance is observed for at least one hardware module 120, such as absence of signals from a temperature sensor, malfunction in electronics and the like. For example, the performance test may be triggered by detection of at least one deviation in heating and/or cooling ramps from any expected profiles, to detect the root cause. For example, the performance test may be triggered in case of high or low heatsink temperature during a run. The performance verification may be automatically triggered and/or executed by at least one control unit. Additionally, the performance verification may be performed upon user-demand, e.g. via at least one user interface.

Figure 1 shows a flowchart in which the method comprises the following exemplary sequence of tests:

    i. (132) basic electric testing of temperature sensors 122;
    ii. (134) testing fan 124;
    iii. (136) testing pump 126;
    iv. (138) consecutively testing of a plurality of thermo-electric cooler pairs 128;
    v. (140) testing power consumption of all thermo-electric coolers 128;
    vi. (142) testing temperature sensor 122 ramp;
    vii. (144) testing vapor chamber mounts 130,
    viii. (146) at least one closing action comprising one or more of disabling all active elements and reporting data.

This order of tests can allow for fulfilling the above-mentioned criteria. However, other orders may be possible.

The method may be performed completely automatically. For example, each of the steps may be executed by the control unit 114, by controlling operation of the respective hardware modules 120. The control unit 114 may be configured for receiving the test results via the communication interface 116. The control unit 114 may be configured for evaluating of the test results and for issuing errors and/or at least one other action.

The method may comprise testing the complete order of steps or aborting the sequence in case of an error occurs.

The flowchart in Figure 1 is depicted in tabular form, wherein in the left column, the method step is shown, in the middle column sub steps comprised by the method steps are shown and in the right column an optional action after the respective method step is depicted such as issuing of an error code or switching the respective hardware module 120 under test of for a subsequent test of a further hardware module 120.

In the embodiment of Figure 1, the basic electric test 132 of temperature sensors 122 may be performed first. The basic electric test 132 may comprise a basic functional test if the temperature sensor 122 or the different temperature sensors 122 are operating at all, e.g. independent from correctness of the measured temperature. In case a temperature value is generated, e.g. a signal from the temperature sensor 122 is received by the control unit 114, the temperature sensor 122 has passed the test. Otherwise, the temperature sensor 122 is erroneous. In this case, an error may be issued and/or noted by the control unit 114. For example, as shown in Figure 1, this basic electric test 132 may comprise the sub steps of reading of all temperature sensors 122 ("Read all sensors (TempAmb)") and defining if all temperature sensors 122 are valid ("Define sensor valid"). In step i. no energy may be inputted or outputted. This step can be performed fast.

In step ii. 134, the fan 124 is tested and the temperature may be reduced. The testing 134 may comprise testing a fan speed. The testing of the fan speed may comprise setting the fan to maximum ("Set FAN On 100 %"), monitoring the fan speed ("Enable/Monitor TIM_ISR (FAN TACHO PIN)"), e.g. by using a fan-internal status signal, and comparing the fan speed to at least one predefined threshold for a maximum fan speed. The threshold may be defined as an expected fan speed according to the fan's datasheet. In case the threshold is exceeded and/or reached, the fan has passed the test. Otherwise, the fan is erroneous. In this case an error may be issued ("FAN_err") and/or noted by the control unit 114. After this test 134, the fan 124 is switched off ("Set Fan OFF 0%").

In step iii. 136 comprises testing pump 126. The temperature may be reduced in this step For example, liquid flow in the cooling circuit may cause energy transfer, therefore, the temperature of the heating block may be reduced in case of increasing temperature of a heatsink (liquid-air heat exchanger). The testing 136 may comprise testing a pump speed. The testing of the pump speed may comprise setting the pump to maximum ("Set PUMP On 100 %"), monitoring the pump speed ("Enable/Monitor TIM_ISR (PUMP TACHO PIN)"), e.g. by using its internal status signal, and comparing the pump speed to at least one predefined threshold for a maximum pump speed. The threshold may be defined as an expected pump speed according to the pump's datasheet. In case the threshold is exceeded and/or reached the pump 126 has passed the test. Otherwise the pump 126 is erroneous. In this case an error ("PUMP_err") may be issued and/or noted by the control unit 114. After this test 136, the pump 126 is switched off ("Set Pump OFF 0%").

Step iv. 138 may comprise consecutively testing of a plurality of thermo-electric cooler pairs 128, e.g. a first TEC pair (TEC1), testing a second TEC pair (TEC2) and testing a third TEC pair (TEC3). For each TEC pair, the testing 138 may comprise measuring current I and/or voltage U during heating and cooling. For example, firstly the heating is set to 100 % ("Set heating 100 %") and current I and/or voltage U are measured ("Measure U and I"). Secondly, the cooling is set of 100 % ("Set cooling 100 %") and current I and/or voltage U are measured ("Measure U and I"). Subsequently, the TEC pair under test is switched off ("SET_TEC_off (0%)"). The testing 138, for each TEC pair, further may comprises comparing the measured current and/or voltage during heating UTTECi_heat (with denoting the respective TEC) and during cooling UTTECi_cool (with denoting the respective TEC) to a predefined threshold ("Threshold"). For example, in Figure 1, the following deviation is calculated:

$$TECDeviation = (\mathrm{UTTECi_{heat}} + \mathrm{UTTECi_{cool}}) / (\mathrm{UTTECj_{heat}} + \mathrm{UTTECj_{cool}}),$$

with i and j denoted the respective TEC of the TEC pair. The threshold may be defined in accordance with electrical parameters of the TEC assuming a worst case scenario, e.g. a worst ambient thermal conditions. In case the threshold is exceeded and/or reached the respective thermo-electric cooler pair has passed the test. Otherwise, it is erroneous. In this case an error ("TECi_err", with denoting the respective TEC) may be issued and/or noted by the control unit 114.

Steps iv. 138 and v. 140 to may be temperature neutral, e.g. the temperature may be increased and reduced.

In step v. 140, the power consumption of all TECs 128 may be tested ("test short circuit"), wherein the temperature is increased. The testing 140 may comprise testing power consumption during heating. The power consumption may be compared to a predefined threshold. The threshold may be defined in accordance with electrical parameters of the TECs assuming a worst case scenario, e.g. a worst ambient thermal conditions. In case the threshold is exceeded and/or reached the thermo-electric coolers 128 have passed the test. Otherwise at least one of them is erroneous. In this case, an error ("Overcurrent_err") may be issued and/or noted by the control unit 114.

In step vi. 142, the testing of the temperature sensor 122 ramp may be performed and the build up temperature of the previous step can be used to measure temperature sensors 122 heating ramp rates. This step 142 may comprise reversing the TEC 128 to cooling and measure cooling ramp rate. As shown in Figure 1, the testing 142 of the response behavior may comprise increasing the temperature to a maximum temperature ("Set_heating 100 % 1.5 s") or decreasing the temperature to a minimum temperature for a predefined time range, e.g. for 1.5 s, reading temperature values generated by the temperature sensor 122 i before changing the temperature and after changing the temperature ("Read all sensors (TempHot)"), calculating a difference *TempDelta(i),* e.g. by

$$TempDelta(i) = (BlockController.TempSensor[i].Temperature - TempOff[i],$$

and comparing the difference *TempDelta(i)* to at least one predefined threshold ("Threshold") for a minimum temperature change. The threshold for a minimum temperature change may be defined with respect to a thermal design of the heating block assuming a worst case scenario, i.e. the worst ambient thermal conditions. In case the threshold for a minimum temperature change is exceeded or at least reached, the temperature sensor 122 i has passed the test. Otherwise, the temperature sensor 122 i is erroneous. In this case an error ("Sensor_err") may be issued and/or noted by the control unit 114.

The testing 144 of the vapor chamber mounts 130 may comprise sequentially controlling the thermo-electric coolers 128 and monitoring of a thermal distribution of the vapor chamber mount 130. The TECs 128 may be spatially arranged between the solid-liquid heat exchanger and the VCM 130. The TECs 128 may be sequentially controlled ("Set TECs 1-2, 5-6 off 1.5 s") and the thermal distribution in the VCM 130 may be monitored ("Read all sensors (TempHot)". The thermal distribution may be compared to a predefined threshold. For example, a mean vaue of the ambient temperature and the hot temperature of all valid temperature sensors may be calculated ("Calc mean val TempAmb and TempHot for all valid TempSensors"). If the number of valid temperature sensors 122 is ≤ 2 an error ("VCM_err") may be issued and/or noted by the control unit 114. Otherwise, a deviation from expected temperature uniformity after a defined time may be calculated, e.g. by

$$TempD(i) = tempHot[i] - TempAmbient[i] + tempMeanValAMB - tempMeanVal,$$

The threshold may be defined as maximum deviation from expected temperature uniformity after a defined time, e.g. as ± 1.5 °C. In case the threshold is not exceeded and/or reached the VCMs have passed the test. Otherwise, at least one of them is erroneous. In this case an error ("VCM err") may be issued and/or noted by the control unit 114. The testing vapor chamber mounts may be performed such that an end temperature is similar to starting temperature of the complete sequence.

Finally, at least one closing action may be performed, e.g. one or more of disabling all active elements and reporting data.

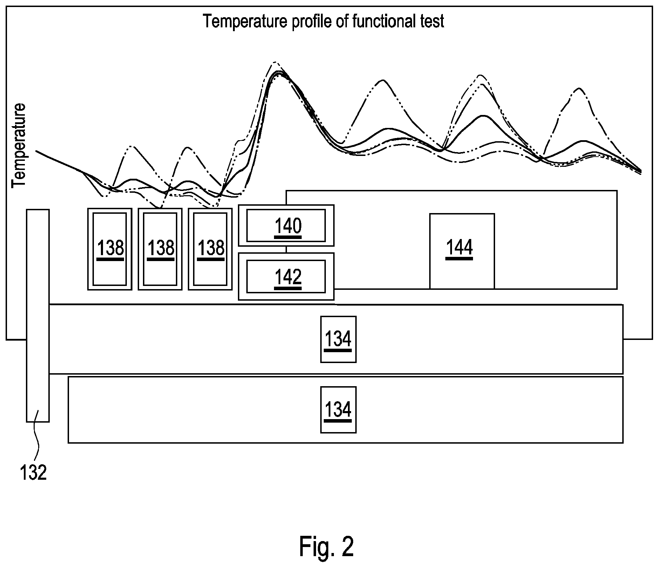

Figure 2 shows experimental data, in particular temperature profiles of five runs of performing the method for performance verification according to Figure 1. This Figure shows that the above-mentioned criteria are fulfilled.

List of reference numbers

[0105]

| | |
|---|---|
| 110 | thermal block cycler unit |
| 112 | laboratory system |
| 114 | control unit |
| 116 | communication interface |
| 118 | thermal block |
| 120 | hardware module |
| 122 | temperature sensor |
| 124 | fan |
| 126 | pump |
| 128 | thermo-electric cooler |
| 130 | vapor chamber mount |
| 132 | basic electric testing |
| 134 | testing fan |
| 136 | testing pump |
| 138 | testing of a plurality of thermo-electric cooler pairs |
| 140 | testing power consumption |
| 142 | testing temperature sensor |
| 144 | testing vapor chamber mounts |
| 146 | closing action |

**Claims**

1. A computer-implemented method for performance verification of a thermal block cycler unit (110) for automated thermal treatment of at least one sample, wherein the thermal block cycler unit (110) comprises at least one thermal block (118) configured for receiving at least one sample vessel,

   wherein the thermal block cycler unit (110) further comprises a plurality of hardware modules (120), wherein the hardware modules (120) comprise a plurality of heating and/or cooling elements and at least one temperature sensor (122),
   wherein the method comprises consecutively functional testing of the hardware modules, wherein an order of tests is defined considering the following criteria

   - every consecutive test uses for testing a thermal energy of its preceding test and/or a status of the thermal block cycler unit (110) of its preceding test, and
   - energy consumption is balanced between the steps in such a way that a difference between an input temperature before consecutively testing the hardware modules (120) and an output temperature after consecutively testing the hardware modules (120) is minimized.

2. The method according to the preceding claim, wherein the hardware modules (120) comprise one or more of at least one temperature sensor (122), at least one fan (124), at least one operational pump (126), at least one at least one thermo-electric cooler (128), at least one vapor chamber mount (130).

3. The method according to the preceding claims, wherein the method comprises testing the temperature sensor (122), wherein the testing comprises an electric test, wherein the electric test comprises testing if a temperature value is generated by the temperature sensor (122).

4. The method according to any one of the two preceding claims, wherein the method comprises testing the temperature sensor (122), wherein the testing comprises testing a response behavior of the temperature sensor (122), wherein the testing of the response behavior comprises increasing the temperature to a maximum temperature or decreasing the temperature to a minimum temperature for a predefined time range, reading temperature values generated by the temperature sensor (122) before changing the temperature and after changing the temperature, calculating a difference and comparing the difference to at least one predefined threshold for a minimum temperature change, wherein in case the threshold is exceeded and/or reached the temperature sensor (122) has passed the test.

5. The method according to any one of the three preceding claims, wherein the method comprises testing the fan (124), wherein the testing comprises testing a fan speed, wherein the testing of the fan speed comprises setting the fan to maximum, monitoring the fan speed, and comparing the fan speed to at least one predefined threshold for a maximum fan speed, wherein in case the threshold is exceeded and/or reached the fan (124) has passed the test.

6. The method according to any one of the four preceding claims, wherein the method comprises testing the operational pump (126), wherein the testing comprises testing a pump speed, wherein the testing of the pump speed comprises setting the pump to maximum, monitoring the pump speed, and comparing the pump speed to at least one predefined threshold for a maximum pump speed, wherein in case the threshold is exceeded and/or reached the pump (126) has passed the test.

7. The method according to any one of the five preceding claims, wherein the method comprises testing the thermo-electric cooler (128), wherein the testing comprises consecutively testing of a plurality of thermo-electric cooler pairs (128), wherein the testing comprises measuring current and/or voltage during heating and cooling, wherein the testing further comprises comparing the measured current and/or voltage during heating and during cooling to a predefined threshold, wherein in case the threshold is exceeded and/or reached the respective thermo-electric cooler (128) pair has passed the test.

8. The method according to any one of the six preceding claims, wherein the method comprises testing all thermo-electric coolers (128), wherein the testing comprises testing power consumption during heating, wherein the power consumption is compared to a predefined threshold, wherein in case the threshold is exceeded and/or reached the thermo-electric coolers (128) have passed the test.

9. The method according to any one of the seven preceding claims, wherein the method comprises testing at least one vapor chamber mount (130), wherein the testing comprises sequentially controlling the thermo-electric coolers (128) and monitoring of a thermal distribution of the vapor chamber mount (130), wherein the thermal distribution is compared to a predefined threshold defined as maximum deviation from expected temperature uniformity after a defined time, wherein in case the threshold is not exceeded and/or reached the vapor chamber mount (130) has passed the test.

10. The method according to any one of the eight preceding claims, wherein the method comprises the following sequence of tests:

    i. (132) basic electric testing of temperature sensors (122);
    ii. (134) testing fan (124);
    iii. (136) testing pump (126);
    iv. (138) consecutively testing of a plurality of thermo-electric cooler pairs (128);
    v. (140) testing power consumption of all thermo-electric coolers (128);
    vi. (142) testing temperature sensor (122) ramp;
    vii. (144) testing vapor chamber mounts (130),
    viii. (146) at least one closing action comprising one or more of disabling all active elements and reporting data.

11. The method according to any one of the preceding claims, wherein the method comprises displaying a result of the performance verification via at least one communication interface (116) and/or at least one user interface, and/or wherein the method comprises reporting the result of the performance verification via the communication interface (116) to a LIMS system.

12. Laboratory system (112) comprising at least one instrument for performing a temperature-dependent reaction comprising a thermal block cycler unit (110) for automated thermal treatment of at least one sample, at least one control unit (114) and at least one communication interface (116), wherein the laboratory system (112) is configured for performing a method for performance verification according to any one of claims 1 to 11.

13. A computer program comprising instructions which, when the program is executed by the laboratory system (112) according to any one of the preceding claims referring to a laboratory system, cause the laboratory system to perform the method according to any one of the preceding claims referring to a method.

14. A computer-readable storage medium comprising instructions which, when the instructions are executed by the laboratory system (112) according to any one of the preceding claims referring to a laboratory system, cause the laboratory system to perform the method according to any one of the preceding claims referring to a method.

15. A non-transient computer-readable medium including instructions that, when executed by one or more processors, cause the one or more processors to perform he method according to any one of the preceding claims referring to a method.

**132** | Read all sensors (TempAmb)
Define sensors valid

**134** | Set Fan ON 100%
Enable/Monitor TIM_ISR (FAN TACHO PIN) → FAN err
Set Fan OFF 0%

**136** | Set Pump ON 100%
Enable/Monitor TIM_ISR (PUMP TACHO PIN) → PUMP err
Set Pump OFF 0%

**138** | Set heating 100%
Measure U and I
Set cooling 100%
Measure U and I
Set TEC off (0%)
$TECDeviation = (UTTEC1\_Heat + UTTEC1\_Cool) / (UTTEC2\_Heat + UTTEC2\_Cool);$
Threshold → TEC1 err

**138** | Set heating 100%
Measure U and I
Set cooling 100%
Measure U and I
Set TEC off (0%)
$TECDeviation = (UTTEC5\_Heat + UTTEC5\_Cool) / (UTTEC6\_Heat + UTTEC6\_Cool);$
Threshold → TEC2 err

Fig. 1

**138**
- Set heating 100%
- Measure U and I
- Set cooling 100%
- Measure U and I
- Set TEC off (0%)
- TECDeviation = (UTTEC3_Heat + UTTEC3_Cool) / (UTTEC4_Heat + UTTEC4_Cool);
- Threshold → TEC3 err

**140**
- Test short circuit → Overcurrent err

**142**
- Set heating 100% 1.5s
- Read all sensors (TempHot)
- TempDelta[i] - BlockController, TempSensor[i], Temperature - TempOff[i];
- Threshold → Sensor err

**144**
- Set TECs 1-2, 5-6 off 1.5s
- Read all sensors (TempHot)
- Calc mean val TempAmb and TempHot for all valid TempSensors
- Valid TempSens <=2 → VCM err
- TempD[i] - (tempHot[i], TempAmbient[i] + tempMeanValAmb - tempMeanVal);
- Threshold (+/-1.5°C) → VCM err

**146**
- Set all TECs off
- Set Fan off
- Set Pump off

( Return OK )    ( Return Err )

Fig. 1
(continued)

Fig. 2

Fig. 3

Europäisches
Patentamt

European
Patent Office

Office européen
des brevets

# EUROPEAN SEARCH REPORT

**Application Number**

EP 23 20 1239

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 2 883 611 A1 (HAIN LIFESCIENCE GMBH [DE]) 17 June 2015 (2015-06-17)<br>* paragraphs [0036], [0044], [0046] – [0050], [0052], [0056], [0063] – [0065] *<br>* figures 1, 2, 3, 4, 9, 10, 11 *<br>----- | 1-15 | INV.<br>B01L7/00<br>G01N35/00 |
| X | US 2014/093947 A1 (MAEDA KOHSHI [JP] ET AL) 3 April 2014 (2014-04-03)<br>* paragraphs [0052] – [0054], [0058], [0059], [0061], [0062], [0064], [0066] – paragraphs [0068], [0080] – [0082], [0108] – [0116], [0120] – [0131], [0149] *<br>* figures 2, 3, 10, 11 *<br>----- | 1-15 | |

| | |
|---|---|
| | **TECHNICAL FIELDS SEARCHED (IPC)** |
| | B01L<br>G01N |

The present search report has been drawn up for all claims

| Place of search | Date of completion of the search | Examiner |
|---|---|---|
| The Hague | 20 March 2024 | Bischoff, Laura |

EPO FORM 1503 03.82 (P04C01)

## ANNEX TO THE EUROPEAN SEARCH REPORT
## ON EUROPEAN PATENT APPLICATION NO.

**EP 23 20 1239**

This annex lists the patent family members relating to the patent documents cited in the above-mentioned European search report.
The members are as contained in the European Patent Office EDP file on
The European Patent Office is in no way liable for these particulars which are merely given for the purpose of information.

20-03-2024

| Patent document cited in search report | | | Publication date | Patent family member(s) | | | Publication date |
|---|---|---|---|---|---|---|---|
| EP 2883611 | A1 | | 17-06-2015 | CN | 105960283 | A | 21-09-2016 |
| | | | | EP | 2883611 | A1 | 17-06-2015 |
| | | | | EP | 3079824 | A1 | 19-10-2016 |
| | | | | ES | 2674944 | T3 | 05-07-2018 |
| | | | | JP | 6524091 | B2 | 05-06-2019 |
| | | | | JP | 2016539648 | A | 22-12-2016 |
| | | | | US | 2016361719 | A1 | 15-12-2016 |
| | | | | US | 2019134638 | A1 | 09-05-2019 |
| | | | | WO | 2015086365 | A1 | 18-06-2015 |
| | | | | ZA | 201603420 | B | 30-08-2017 |
| US 2014093947 | A1 | | 03-04-2014 | CN | 103635568 | A | 12-03-2014 |
| | | | | DE | 112012002529 | T5 | 27-02-2014 |
| | | | | JP | 5703377 | B2 | 15-04-2015 |
| | | | | JP | WO2012176596 | A1 | 23-02-2015 |
| | | | | US | 2014093947 | A1 | 03-04-2014 |
| | | | | WO | 2012176596 | A1 | 27-12-2012 |

EPO FORM P0459

For more details about this annex : see Official Journal of the European Patent Office, No. 12/82

**EP 4 534 205 A1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2022101870 A1
- US 20140122006 A1
- US 5602756 A
- EP 2108942 A1
- US 10850283 B2
- US 20130157376 A1
- KR 2375707 B1
- EP 3253492 A2
- US 6703236 B2
- AU 741806 B2
- US 20080212643 A1
- EP 1710017 A1
- WO 2013075839 A